# EUROPEAN PATENT APPLICATION

(11) **EP 0 571 038 A1**
(43) Date of publication of application: **24.11.1993**
(21) Application number: 93201405.3
(22) Date of filing: 18.05.1993
(51) Int. Cl.: C07D 251/34, C08G 18/79

(54) **Process for the preparation of a polyisocyanate containing isocyanurate groups**

(30) Priority: 22.05.1992 NL 9200906
(71) Applicant: DSM N.V., NL-6411 TE Heerlen (NL)
(72) Inventor: Frentzen, Yvonne Helene, NL-5921 EL Venlo (NL); Hendrix, Jan Agnes Jozef, NL-6125 CB Born (NL); Kresta, Jiri Eric, 48092 Warren, Michigan (US); Youlu, Duan, Minneapolis, MN55454 (US)

(57) **Abstract**

The invention relates to a process for the preparation of a compound containing polyisocyanate and isocyanurate groups through trimerisation of 1,4-tetramethylenediisocyanate (TMDI), using a trimerisation catalyst, and the purification of the trimer desired, characterised in that the trimerisation is effected at a temperature below 90°C, the purification being effected through extraction, using an apolar solvent with a boiling point below 180°C, and evaporation of the solvent under lowered pressure, at a temperature below 90°C.

## Description

The invention relates to a process for the preparation of a compound containing polyisocyanate and isocyanurate groups through trimerisation of 1,4-tetramethylenediisocyanate (TMDI), using a trimerisation catalyst, and purification of the trimer desired.

Such a process is described in EP-A-273836, which describes trimerisation of a diisocyanate and that trimers of diisocyanates can be purified by treating a reaction mixture with supercritical carbon dioxide. However, such a purification step requires relatively expensive equipment in view of the high pressures required. On the other hand, the methods usually used - and also in part in EP-A-273836 - for the purification of trimers involves evaporation of the monomer under lowered pressure. These are not suitable for TMDI.

The aim of the invention is to provide a process according to which a good yield of the desired trimer is obtained and according to which the desired trimer can be obtained in a pure form using a simple purification step.

This aim is achieved according to the invention as the trimerisation is carried out at a temperature below 90°C, the purification being effected through extraction using an apolar solvent with a boiling point below 180°C and evaporation of residual solvent from the trimer under reduced pressure, at a temperature below 90°C.

From EP-A-105242 it is known to add an apolar solvent, in an amount of between 2 and 30 wt.%, relative to the trimer, to a mixture of trimer and a small amount of monomer, obtained after partial evaporation of the monomer. The solvent is then evaporated along with a portion of the residual monomer. This process involves no extraction and use is made of only relatively small amounts of apolar solvent.

The trimerisation can be carried out in a solvent. Suitable solvents are alkylesters with 3-8 carbon atoms, such as ethylacetate, methylacetate, t-butylacetate, n-butylacetate, methylversatate, methylproprionate and methylbutyrate. It is however preferable to use no solvent.

If use is made of a solvent, the amount of TMDI monomer is generally between 10 and 200 wt.% relative to the solvent, preferably between 50 and 150 wt.%
The trimerisation reaction is carried out at a temperature below 90°C. At a higher temperature polymer material appears to be formed. Preferably, the reaction temperature is chosen between 50 and 75°C.

As catalyst use is generally made of a catalyst that is relatively active with respect to the trimerisation. Very suitable are for example quaternary ammonium salt in ethylene glycol (Dabco TMR^{R,} from Air Products), a tertiary amine glycol mixture (Dabco 798^{R}, from Air Products), 1,3,5-tris-(dimethylaminopropyl)hexahydrotriazin (Polycat 41^{R}, from Air Products), a mixture of amide and glycol containing dimethylformamide (Curithane 51^{R}, from Air Products) or an amine in glycol (Curithane 52^{R}, from Air Products).

The diluted catalyst is preferably added in small portions during the reaction. The total amount of catalyst can be simply determined by a person skilled in the art and is generally between 3 and 0.01 g per kg of TMDI, preferably between 1 and 0.01 g. The catalyst can be added in 2-5 times, and is preferably added in 3-4 times.

The trimerisation is usually stopped at a degree of conversion of between 50 and 90%. Higher degrees of conversion may result in oligomeric and polymeric reaction products, while lower degrees are less attractive from an economic point of view. Preferably, the reaction is stopped at a degree of conversion of between 60 and 80%.

The reaction is stopped by adding an inhibitor that is suitable for the catalyst system: usually an organic or inorganic acid, such as monochloroacetic acid, paratoluenesulphonic acid or phosphoric acid.

After inhibition, the trimer is purified by removing the monomer in particular through repeated extraction using an apolar solvent. The apolar solvent has a boiling point (at atmospheric pressure) below 180°C. Preferably, the solvent has a boiling point between 50 and 140°C, more in particular between 50 and 110°C. Aliphatic hydrocarbons with between 5 and 8 carbon atoms are very suitable, for example pentane, cyclopentane, n-hexane, cyclohexane, chloroform, trichloroethane, 2-methylpentane, 2-methylhexane, n-heptane, n-octane and 2-ethylhexane. Also very suitable are aromatic hydrocarbons with between 6 and 8 carbon atoms such as benzene, toluene or xylene and ethers with between 4 and 8 carbon atoms such as diethylether, dipropylether and ethyl-t-butylether. Preferably, an aliphatic hydrocarbon is chosen for the extraction because the trimer is virtually insoluble in it.

The monomer appears to dissolve in the extraction agent, whereas the trimer remains as a separate phase. Via repeated extraction it is easy to obtain a product with a monomer content of less than 3 wt.%, preferably less than 1 wt.%, relative to the trimer. The residual solvent -if any-is then removed from the trimer through evaporation at a temperature below 90°C. The evaporation is preferably done at a temperature between 20 and 75°C, and at lowered pressure.

Such a pure product is novel. TMDI is frequently mentioned as a monomer to be trimerised in the state of the art, but it is impossible to obtain the trimer in a pure form (containing less than 3 wt.%, preferably less than 1 wt.% monomer relative to the trimer) using the purification methods described there. The trimer also appears to have surprisingly good properties when used as a cross-linking agent in a coating or paint resin. The hardness of the coating or paint in particular has been found to increase.

Such a product is very suitable for use as a cross-linking component in polyurethane coatings. To this end it may also be desirable to block the isocyanate groups present in the trimer using for example alcohols such as t-butanol, secondary amines such as N-methylaniline and lactams such as ε-caprolactam. Also phenol and methyl-ethylketoxim are well suited.

The process will be elucidated with reference to the following non-restrictive examples.

### Example I

10 g of 1,4-butanediisocyanate (tetramethylene diisocyanate, TMDI) was heated to 60°C under a nitrogen blanket, in a 100-ml three-necked flask. After half an hour 1 mg of Polycat 41^{R} (1% in n-hexane) was added to the TMDI. After half an hour the same amount of Polycat^{R} was added and then again after 6.5 hours. The reaction was stopped at an NCO value (isocyanate value) of 39.2% (as determined via back-titration of di-n-butylamine). As the monomer has an NCO value of 60 (60 wt.% of the pure compound consists of NCO) and the trimer has an NCO value of 30, the value of 39.2% implies a 69% degree of conversion. The reaction was stopped by adding 3 mg of monochloroacetic acid, after which the mixture was stirred at 60°C for half an hour. Then the mixture was cooled to room temperature and was extracted three times using n-hexane (the reaction mixture/hexane volumetric ratio was 1:4). The liquid trimer was separated and a small amount of residual solvent was removed through evaporation at 40°C, under a lowered pressure. The trimer yield amounted to 60%; the isocyanate value was 28.7%. The 1690 cm⁻¹/2270 cm⁻¹ IR peak ratio was 0.51. The monomer content was lower than 1 wt.%.

### Example II

TMDI was trimerised analogously to example I only now use was made of 1 g of TMDI and 5 g of butylacetate. Dabco TMR^{R} was used as a catalyst. After extraction using n-hexane and evaporation of the residual n-hexane the desired trimer had an isocyanate value of 29.5%. The monomer content was lower than 1 wt.%.

### Example III

A trimer was obtained with low monomer content according to the method of example II, using 5 g of TMDI in 5 g of butylacetate and Curithane 51^{R} as a catalyst.

## Claims

1. Process for the preparation of a compound containing polyisocyanate and isocyanurate groups through trimerisation of 1,4-tetramethylenediisocyanate (TMDI) at a temperature below 90°C, using a trimerisation catalyst, and purification of the trimer desired, which purification is effected through extraction, using an apolar solvent with a boiling point below 180°C, and evaporation of residual solvent from the trimer under lowered pressure, at a temperature below 90°C.

2. Process according to claim 1, characterised in that the trimerisation is carried out at a temperature between 50 and 75°C.

3. Process according to claim 1 or claim 2, characterised in that the extraction is effected using aliphatic hydrocarbons with between 5 and 8 carbon atoms, aromatic hydrocarbons with between 6 and 8 carbon atoms or ethers with between 4 and 8 carbon atoms.

4. Process according to any one of claims 1-3, characterised in that the solvent used for the extraction has a boiling point of between 50 and 140°C.

5. TMDI trimer obtainable with the aid of a process according to any one of claims 1-4, containing less than 3 wt.% TMDI monomer, relative to the trimer.

6. TMDI trimer according to claim 5, containing less than 1 wt.% TMDI monomer, relative to the trimer.

7. Use of a TMDI trimer according to any one of claims 5-6, optionally with blocked isocyanate groups, as an isocyanate component in polyurethane coatings.
